# DEMANDE DE BREVET EUROPEEN

(11) **EP 3 320 871 A1**
(43) Date de publication de la demande: **16.05.2018**
(21) Numéro de dépôt: 17306405.6
(22) Date de dépôt: 17.10.2017
(51) Int. Cl.: A61B 17/88, A61B 17/82, A61B 17/86, A61B 17/04

(54) **ÉQUIPEMENT POUR LE TRAITEMENT D'UNE FRACTURE PAR HAUBANAGE, PAR EXEMPLE D'UNE FRACTURE DE L'OLÉCRANE OU DE LA ROTULE**

(30) Priorité: 19.10.2016 FR 1660139
(71) Demandeur: Newclip International, 2163 Luxembourg (LU)
(72) Inventeur: LE GUILLOUX, Pierre, 83000 TOULON (FR); LARCHE, Grégoire, 49300 CHOLET (FR); PODGORSKI, Jean-Pierre, 49230 St-CRESPIN SUR MOINE (FR)
(74) Mandataire: Jacobacci Coralis Harle

(57) **Abrégé**

L'invention concerne un équipement (1) pour le traitement d'une fracture par haubanage, par exemple d'une fracture de l'olécrane ou de la rotule, lequel équipement (1) comprend au moins deux broches de maintien (2) associées à un câble de haubanage (4), chaque broche de maintien (2) comportant un corps allongé (21) destiné à être enfoncé dans le matériau osseux de réception (R), lequel corps allongé (21) comporte une extrémité distale qui se termine par une pointe (23), et une extrémité proximale qui se termine par une tête (25).
Selon l'invention, ledit corps allongé (21) desdites broches de maintien (2) comporte au moins une partie (211) munie d'un filetage, ladite tête (25) desdites broches de maintien (2) comportant un orifice (252) destiné au passage dudit câble de haubanage (4) ;
et ledit équipement (1) comprend encore un outil de manoeuvre (3) muni d'une tête de travail (32) agencée pour coopérer avec la tête (25) d'une desdites broches de maintien (2, 2'), pour la manoeuvre en rotation de son corps allongé (21) autour de son axe longitudinal, en vue de son ancrage par vissage dans ledit matériau osseux de réception (R) au moyen de ladite partie filetée (211).

## Description

### DOMAINE TECHNIQUE AUQUEL SE RAPPORTE L'INVENTION

La présente invention concerne le domaine général des équipements utilisés pour la mise en oeuvre de techniques de chirurgie osseuse.
Elle concerne plus particulièrement un équipement d'ostéosynthèse pour le traitement d'une fracture par haubanage, par exemple d'une fracture de l'olécrane ou de la rotule.

### ARRIERE-PLAN TECHNOLOGIQUE

Le traitement d'une fracture de l'olécrane ou de la rotule peut être réalisé, après réduction, par une technique dite de « haubanage », au moyen d'un équipement comprenant au moins deux broches de maintien associées à un cerclage de haubanage généralement en câble d'acier.

Les broches de maintien comprennent chacune un corps allongé lisse muni d'une extrémité distale acérée, et d'une extrémité proximale qui se termine par une tête.

Chaque broche est enfoncée dans l'os par son extrémité acérée, au moyen d'un moteur chirurgical, selon une orientation judicieusement choisie par le chirurgien.

Un cerclage est réalisé à l'aide d'un câble de haubanage reposant sur les fragments osseux et sur les broches préalablement positionnées. Les extrémités du câble sont ensuite reliées et torsadées afin de tendre le montage et de réaliser une compression entre les différents fragments à assembler.
Les broches sont ensuite raccourcies, recourbées puis enfouies.

Cependant, au fil du temps, sous l'action des gestes et des sollicitations répétées du membre fracturé, il arrive que les broches migrent au sein de l'os de réception, dans le sens d'un recul.
Dans ce cas, la migration secondaire des broches entraine des lésions cutanées avec des risques d'infection du site opératoire.
Par ailleurs, la tension sur le câble de haubanage diminue, ce qui provoque une baisse de la stabilité du pontage, une déficience de la compression du foyer de fracture, et une perte significative de la correction orthopédique. Ces complications peuvent être la source de pseudarthrose ou de cal vicieux, avec risque de reprise chirurgicale.

### OBJET DE L'INVENTION

La présente invention a pour but de remédier à ces inconvénients en proposant un équipement facile à poser, qui permet d'éviter les problèmes de migration secondaire, d'assurer une compression de qualité sur la fracture traitée, et durable dans le temps, afin de faciliter la fonction du membre.

Pour cela, l'équipement selon l'invention pour le traitement d'une fracture par haubanage est du type comprenant au moins deux broches de maintien associées à un câble de haubanage, chaque broche de maintien comportant un corps allongé, d'axe longitudinal L, destiné à être enfoncé dans le matériau osseux de réception, lequel corps allongé comporte une extrémité distale qui se termine par une pointe, et une extrémité proximale qui se termine par une tête ; et conformément à l'invention :
- ledit corps allongé desdites broches comporte au moins une partie munie d'un filetage,
- ladite tête desdites broches comporte un orifice adapté au passage dudit câble de haubanage,
- et ledit équipement comprend encore un outil de manoeuvre, muni d'une tête de travail agencée pour coopérer avec la tête d'une desdites broches, pour la manoeuvre en rotation de son corps allongé autour dudit axe longitudinal L, en vue de son ancrage par vissage dans le matériau osseux de réception au moyen de ladite partie filetée.

L'orifice ménagé dans la tête de broche facilite le positionnement et le maintien du câble de haubanage. Cette tête de broche participe également à l'enfoncement du corps de la broche dans le matériau osseux de réception, au moyen de l'outil de manoeuvre, pour obtenir un positionnement stable et durable dans le temps.

D'autres caractéristiques non limitatives et avantageuses de l'équipement conforme à l'invention, prises individuellement ou selon toutes les combinaisons techniquement possibles, sont les suivantes :
- la tête des broches est en forme d'anneau muni d'une embase raccordée de façon monobloc avec l'extrémité proximale de leur corps allongé, laquelle tête en forme d'anneau comporte ledit orifice de passage du câble de haubanage et est délimitée par deux faces latérales, par deux faces d'extrémités et par une face de dessus ;
- ladite tête en forme d'anneau est raccordée avec l'extrémité proximale du corps allongé par l'intermédiaire d'une structure de butée monobloc, pour bloquer son enfoncement dans le matériau osseux de réception ;
- la tête de travail de l'outil de manoeuvre comporte un logement débouchant destiné à la réception de ladite tête en forme d'anneau de chacune desdites broches, lequel logement débouchant comprend deux surfaces de côtés, en vis-à-vis, entre lesquelles ladite tête en forme d'anneau est destinée à venir se positionner, avec ses faces latérales en regard desdites surfaces de côtés, pour permettre la transmission d'un couple en vue de la manoeuvre en rotation du corps allongé de chacune desdites broches, pour son ancrage par vissage dans le matériau osseux de réception ;
- ladite tête de travail de l'outil de manoeuvre et ladite tête de chacune desdites broches comportent des moyens complémentaires pour leur assemblage amovible, de préférence par emboitement élastique ;
   dans ce cadre, le logement débouchant de la tête de travail dudit outil de manoeuvre est avantageusement bordé par une paroi latérale comportant deux fenêtres en vis-à-vis qui s'ouvrent dans ledit logement débouchant, pour le positionnement, chacune, d'une partie active d'un anneau élastique ceinturant ladite paroi latérale ; et la tête en forme d'anneau de chacune desdites broches comporte deux échancrures ménagées dans lesdites faces d'extrémités, pour la réception desdites parties actives dudit anneau élastique, en fin d'introduction de ladite tête en forme d'anneau dans ledit logement débouchant dudit outil de manoeuvre, pour obtenir ledit emboitement élastique amovible ;
- l'orifice de passage du câble de haubanage ménagé dans la tête desdites broches est de forme oblongue pour autoriser un jeu de positionnement pour ledit câble de haubanage ;
- la tête desdites broches est structurée pour autoriser sa déformation par écrasement sur le câble de haubanage ;
- dans un mode de réalisation particulier, le corps allongé d'au moins une desdites broches comporte deux parties filetées séparées par une âme non filetée, lesquelles deux parties filetées comportent un pas de vis différent pour autoriser une mise en compression de deux parties du matériau osseux de réception séparées par une fracture.

### DESCRIPTION DETAILLEE D'UN EXEMPLE DE REALISATION

L'invention sera encore illustrée, sans être aucunement limitée, par la description suivante d'une forme de réalisation particulière, donnée uniquement à titre d'exemple et représentée sur les dessins annexés dans lesquels :
- la figure 1 est une vue de dessus schématique d'une rotule de genou fracturée, traitée par un équipement d'ostéosynthèse conforme à la présente invention ;
- la figure 2 est une vue de côté de la rotule fracturée illustrée sur la figure 1 ;
- la figure 3 est une vue de face d'un mode de réalisation possible d'une broche de maintien faisant partie de l'équipement d'ostéosynthèse selon l'invention ;
- la figure 4 est une vue de côté de la broche de maintien illustrée sur la figure 3;
- la figure 5 est une vue de dessus de la broche illustrée sur les figures 3 et 4 ;
- la figure 6 est une vue de face d'un second mode de réalisation possible d'une broche de maintien faisant partie de l'équipement d'ostéosynthèse selon l'invention ;
- la figure 7 est une vue de côté de la broche de maintien illustrée sur la figure 6;
- la figure 8 est une vue schématique, en perspective, d'un outil de manoeuvre faisant partie de l'équipement d'ostéosynthèse selon l'invention, pour la manoeuvre en rotation des broches de maintien ;
- la figure 9 est une vue agrandie, en perspective, de la tête de travail de l'outil de manoeuvre illustré sur la figure 8 ;
- la figure 10 est une vue de côté de l'outil de manoeuvre illustré sur les figures 8 et 9;
- la figure 11 est une vue en coupe de l'outil de manoeuvre des figures 8 à 10, selon le plan de coupe 11-11 de la figure 10 ;
- la figure 12 est une vue agrandie de la tête de travail de l'outil de manoeuvre illustré en coupe sur la figure 11 ;
- la figure 13 est une vue de côté qui illustre le montage d'une broche de maintien selon les figures 6 et 7 sur la tête de travail de l'outil de manoeuvre illustré sur les figures 8 à 12, pour la pose par vissage de ladite broche de maintien dans le matériau osseux de réception ;
- la figure 14 est une vue en coupe selon le plan de coupe 14-14 de la figure 13;
- la figure 15 est une vue agrandie d'une partie de la figure 14, illustrant le détail du montage de la tête de broche dans la tête de travail de l'outil de manoeuvre.

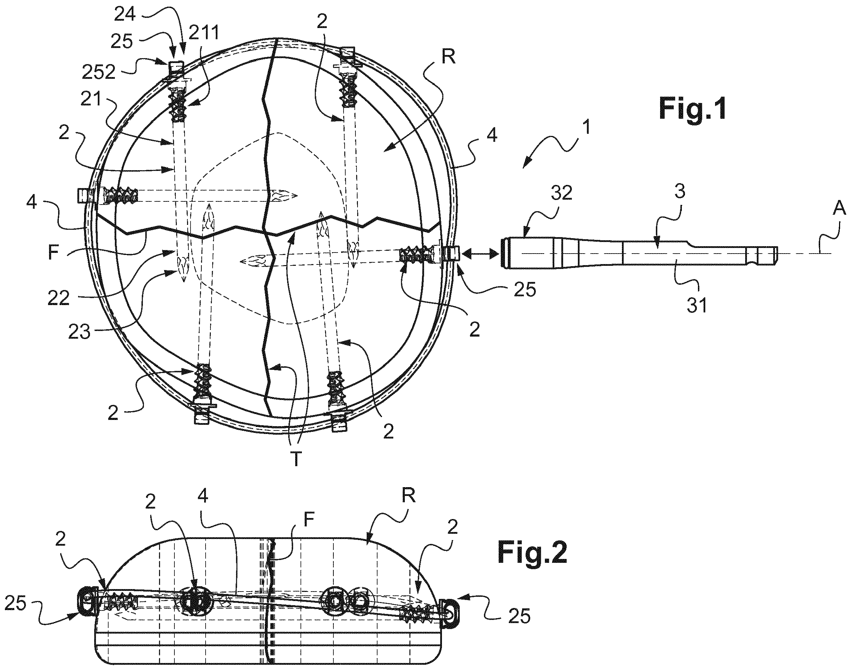

Sur les figures 1 et 2, on a représenté schématiquement un matériau osseux, en l'occurrence une rotule de genou R, comportant une fracture F, composée en l'occurrence de plusieurs traits de fracture, traitée par un équipement chirurgical d'ostéosynthèse 1 selon l'invention.

Cet équipement 1 comprend :
a/ au moins deux broches de maintien 2 (en l'occurrence ici au nombre de six), comportant - une tête munie d'un orifice, et - un corps dont au moins une partie est munie d'un filetage, chacune de ces broches de maintien 2 étant adaptée à être enfoncée et vissée dans le matériau osseux R, au travers des traits de fracture F pour aligner les différents fragments osseux dans une position dite anatomique ;
b/ un outil de manoeuvre 3 (non représenté sur la figure 2), adapté pour enfoncer par vissage les broches de maintien 2 dans le matériau osseux de réception R, et
c/ un câble de haubanage 4, qui est adapté pour passer au travers des orifices des têtes des broches de maintien 2, pour guider son circuit, et qui est agencé pour ceinturer la zone fracturée afin de maintenir les fragments osseux assemblés par les broches.

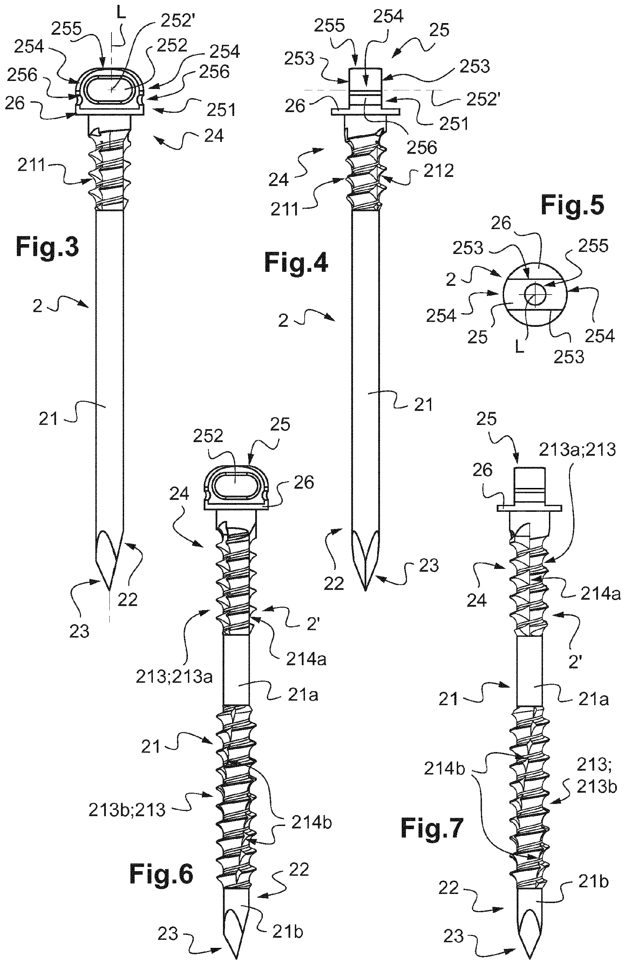

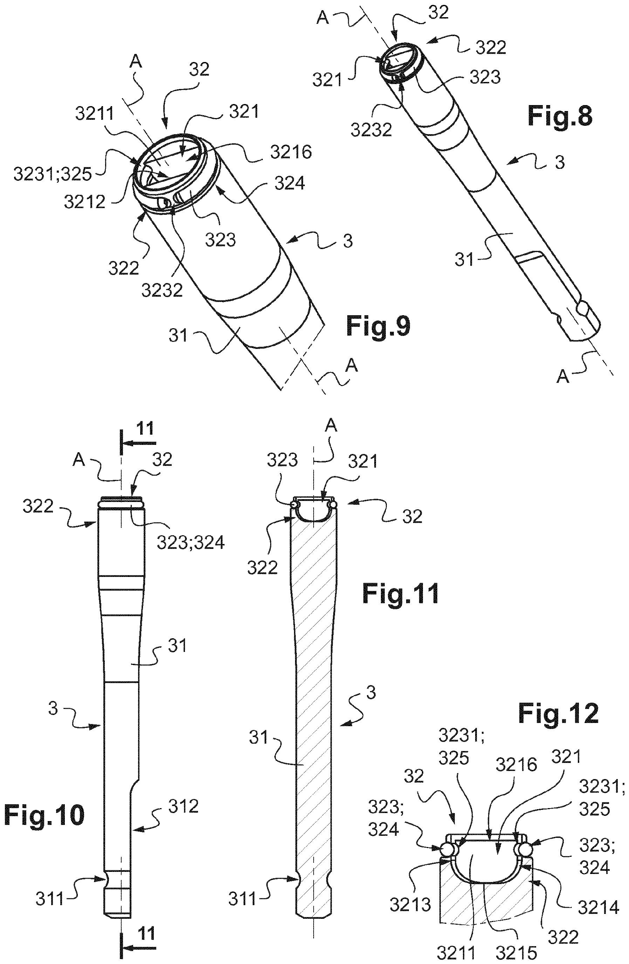

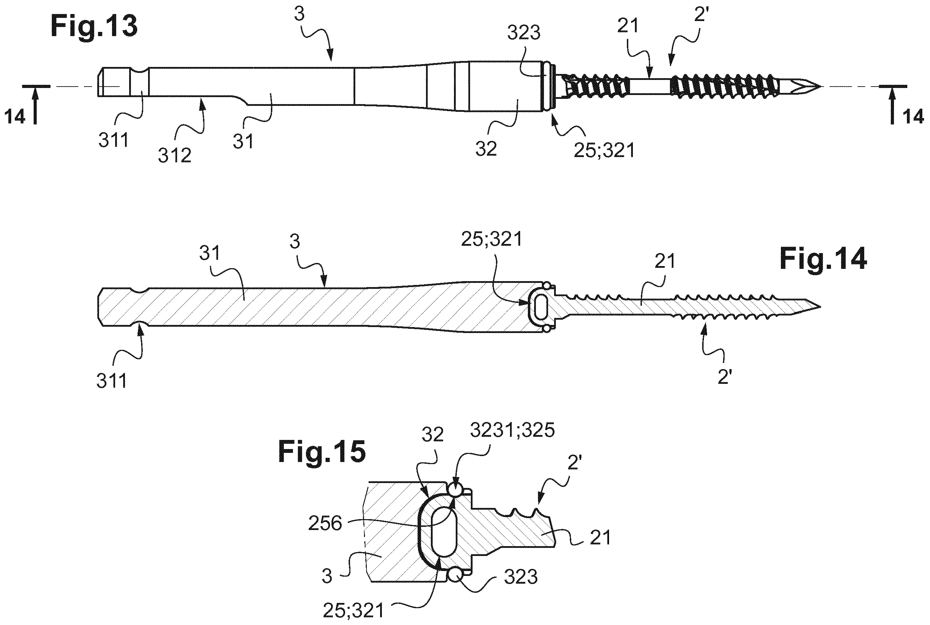

Une des broches de maintien 2 est représentée isolément sur les figures 3 à 5.
Une variante de réalisation possible de ces broches de maintien 2' est illustrée sur les figures 6 et 7.
L'outil de manoeuvre 3 est représenté isolément sur les figures 8 à 12 ; et la coopération entre cet outil de manoeuvre 3 et une des broches de maintien 2' est illustrée sur les figures 13 à 15.

Comme on peut le voir sur les figures 3 à 5, chaque broche de maintien 2 comporte un corps allongé 21 d'axe longitudinal L destiné à être enfoncé dans le matériau osseux de réception R ; l'extrémité distale 22 de ce corps allongé 21 se termine par une pointe 23, et son extrémité proximale 24 se termine par une tête 25.

La broche de maintien 2 est réalisée monobloc en tout matériau approprié (par exemple titane, acier inoxydable ou matériau résorbable).

Son corps allongé 21 a une forme générale cylindrique dont le diamètre est par exemple compris entre 1,5 et 3 mm, sa longueur totale pouvant être comprise entre 10 mm et 100 mm (de préférence entre 15 et 60 mm).

La tête 25 de la broche 21 se présente sous la forme d'un anneau muni d'une embase 251 raccordée de façon monobloc avec l'extrémité proximale 24 du corps allongé 21.

Cette tête 25 comporte un orifice 252 dont l'axe 252' s'étend perpendiculairement à l'axe longitudinal L du corps de vis 21, pour le passage du câble de haubanage 4. Elle présente une forme globalement parallélépipédique à grande section rectangulaire, délimitée par deux faces latérales 253, par deux faces d'extrémités 254 et par une face de dessus 255.

Les deux faces latérales 253 sont parallèles entre elles et elles définissent l'épaisseur de la tête 25, qui peut être de l'ordre de 2 à 4 mm. Les faces d'extrémités 254 sont sensiblement parallèles entre elles et la distance qui les sépare définit la longueur de la tête 25. Les deux angles qui raccordent les faces d'extrémités 254 et la face de dessus 255 sont arrondis en particulier pour limiter l'agressivité au regard des tissus environnants après implantation.

L'orifice 252 de la tête 25 est dimensionné pour permettre le passage du câble de haubanage 4 ; il est ici de forme oblongue avec son grand axe orienté perpendiculairement à l'axe longitudinal L, de façon à permettre un jeu de positionnement pour ledit câble de haubanage 4.

Sur les figures 3 et 4, on remarque la présence de deux échancrures 256, ménagées en opposition dans les faces d'extrémités 254 de la tête 25, au niveau de son embase 251. Chacune de ces échancrures 256 est en forme d'arc de cercle centré sur l'axe longitudinal L. Le diamètre du cercle passant par le fond de ces deux échancrures 256 est légèrement inférieur à la longueur de la tête 25.
La fonction de ces échancrures 256 sera expliquée plus loin.

Le corps allongé 21 de la broche de maintien 2 comporte une partie munie d'un filetage 211 pour permettre son ancrage par vissage dans le matériau osseux de réception R.

Dans le mode de réalisation illustré sur les figures 3 à 5, cette partie filetée 211 s'étend au niveau de la partie proximale 24 du corps allongé 21.

Le filet de cette partie filetée 211 comporte trois rainures d'autotaraudage rectilignes 212.

Entre cette partie filetée 211 et la pointe 23, le corps allongé 21 est lisse, dépourvu de filetage.

La partie proximale 24 du corps allongé 21 est raccordée à l'embase 251 de la tête 25 par l'intermédiaire d'une structure de butée monobloc 26, adaptée pour bloquer l'enfoncement de la broche de maintien 2 dans le matériau osseux de réception R.

Cette butée monobloc 26 est en forme de pastille circulaire centrée sur l'axe longitudinal L de la broche de maintien 2, et qui s'étend dans un plan perpendiculaire à ce dernier.
Cette butée monobloc 26 déborde sur le pourtour du corps allongé 21 ; son diamètre peut être de l'ordre du double de celui dudit corps allongé 21, ce diamètre correspondant approximativement à la longueur de la tête 25.

Les figures 6 et 7 illustrent une variante de réalisation d'une broche 2' de l'équipement 1 selon l'invention.

Les parties identiques ou similaires à la broche 2 illustrée sur les figures 3 à 5 conservent les mêmes repères par mesure de simplification.

Cette broche de maintien 2' se distingue de la broche de maintien 2 décrite ci-dessus uniquement par la structure du filetage de son corps allongé 21.

Le corps allongé 21 de cette broche de maintien 2' comporte un filetage 213 constitué de deux parties filetées 213a et 213b séparées par une âme lisse non filetée 21a.

Une première partie filetée 213a s'étend au niveau de la partie proximale 24 du corps allongé 21, de manière similaire à la partie filetée 211 de la broche de maintien 2 des figures 3 à 5.

L'âme lisse 21a s'étend dans la partie centrale du corps allongé 21.

La seconde partie filetée 213b s'étend entre l'âme lisse 21a et la partie distale 22 du corps allongé 21.

Une partie terminale lisse 21b non filetée est préservée entre cette seconde partie filetée 213b et la pointe 23 du corps allongé 21.

Le filet de la partie filetée 213a comporte trois rainures d'autotaraudage 214a rectilignes ; de son côté, le filet de la partie filetée 213b comporte trois rainures d'autotaraudage 214b de type hélicoïdal.

Les pas de vis des filets des deux parties filetées 213a et 213b sont différents pour assurer une mise en compression de deux parties du matériau osseux de réception R, séparées par une fracture F.

L'outil de manoeuvre 3, visible sur la figure 1, est illustré de manière isolée sur les figures 8 à 12.

Cet outil de manoeuvre 3 comporte un corps longitudinal 31 de forme générale cylindrique, dont l'une des extrémités se termine par une tête de travail 32.

Le corps longitudinal 31, d'axe longitudinal A, constitue un manche pour la prise en main de l'outil de manoeuvre 3 par un opérateur (en particulier un chirurgien) ; et la tête de travail 32 est adaptée pour coopérer avec la tête 25 d'une broche de maintien 2, 2' pour la manoeuvre en rotation de son corps allongé 21 autour de son axe longitudinal L, en vue de son ancrage par vissage dans le matériau osseux de réception R au moyen de sa partie filetée 211, 213 (et aussi pour son dévissage éventuel).

Pour cela, cette tête de travail 32 comporte un logement débouchant 321, de forme complémentaire à celle de la tête 25 des broches de maintien 2, 2', pour permettre la réception de cette tête 25 et son entrainement en rotation par la manoeuvre en rotation du corps longitudinal 31.

Le logement débouchant 321 est ménagé au sein de la tête de travail 32, dans le prolongement du corps longitudinal 31 ; il est centré sur l'axe longitudinal A.

Ce logement débouchant 321 présente une forme globalement parallélépipédique, et il comporte - deux surfaces de côtés 3211 et 3212 en vis-à-vis, parallèles entre elles, - deux surfaces d'extrémités 3213 et 3214, parallèles ou sensiblement parallèles entre elles, ainsi qu'- une surface de fond 3215.

La bordure d'extrémité des surfaces de côtés 3211 et 3212 et des deux surfaces d'extrémités 3213 et 3214 définit l'ouverture 3216 du logement débouchant 321.
Cette ouverture 3216 est de forme générale rectangulaire, centrée sur l'axe longitudinal A de l'outil de manoeuvre 3.

Le logement débouchant 321 est bordé par une paroi latérale 322 qui prolonge le corps cylindrique 31. Cette paroi latérale 322 est de forme tubulaire dont la surface interne délimite le logement débouchant 321 et comporte les surfaces de côtés 3211, 3212, d'extrémités 3213, 3214 et de fond 3215, et dont la surface extérieure est de forme générale cylindrique.

Le logement débouchant 321 de la tête de travail 32 de l'outil de manoeuvre 3 peut donc venir coiffer la tête 25 des broches de maintien 2, 2'.
Alors, les faces d'extrémités 254 de la tête 25 des broches de maintien 2, 2' viennent se positionner en regard des surfaces d'extrémités 3213 et 3214 du logement débouchant 321 ; et les faces latérales 253 de la tête de broche 25 viennent se positionner en regard des surfaces de côtés 3211, 3212 du logement débouchant 321, cela pour permettre la transmission d'un couple, en vue de la manoeuvre en rotation du corps allongé 21 de la broche 2, 2' pour l'ancrage par vissage dans le matériau osseux de réception R (et aussi pour le dévissage).

De manière à faciliter les opérations de vissage et de dévissage par l'opérateur, la tête de travail 32 de l'outil de manoeuvre 3, et la tête 25 des broches de maintien 2, 2' comportent des moyens complémentaires pour leur assemblage amovible.

Ces moyens d'assemblage amovible se présentent sous la forme de moyens d'assemblage par emboitement élastique consistant ici en un anneau élastique 323 agencé pour coopérer avec les échancrures précitées 256 ménagées dans les faces d'extrémités 254 de la tête de vis 25.

L'anneau élastique 323 consiste en un anneau circulaire ouvert, par exemple en métal, qui est logé dans une rainure d'accueil 324 ménagée à l'extrémité de la tête 32, sur le pourtour extérieur de la paroi latérale 322, centrée sur l'axe longitudinal A.
La section de l'anneau élastique 323 est de forme circulaire.

Au niveau de la rainure d'accueil 324, la paroi latérale 322 comporte deux fenêtres en vis-à-vis 325 qui débouchent dans le logement 321 pour le positionnement chacune d'une partie active 3231 de l'anneau élastique 323.

Les fenêtres en vis-à-vis 325 sont ici ménagées dans les surfaces d'extrémités 3213 et 3214 du logement débouchant 321 ; et les parties actives 3231 de l'anneau élastique 323 font saillie dans le logement débouchant 321, au niveau de ces surfaces d'extrémités 3213 et 3214, avec la possibilité de s'escamoter dudit logement 321 par une poussée vers l'extérieur du fait de ses caractéristiques d'élasticité.
L'ouverture 3232 de l'anneau élastique 323 est visible sur les figures 8 et 9.

Le corps longitudinal 31 de l'outil de manoeuvre 3 comporte des moyens en forme de gorge 311 et de méplat 312, classiques, pour son montage sur un moteur chirurgical manipulé par l'opérateur.

Les figures 13 à 15 illustrent l'assemblage amovible entre une broche de maintien 2' et l'outil de manoeuvre 3, par emboitement élastique de la tête de broche 25 dans le logement débouchant 321 dudit outil 3.

Comme on peut le voir sur les figures 13 à 15, la tête 25 des broches de maintien 2, 2' vient s'insérer dans le logement 321 par l'ouverture d'extrémité 3216 de ce dernier. Les deux parties actives 3231 de l'anneau élastique 323 font saillie au niveau des emplacements des échancrures 256 de la tête 25 des broches de maintien 2, 2', lorsque cette tête 25 est complètement insérée dans le logement débouchant 321 de l'outil de manoeuvre 3 ; et elles sont adaptées pour obtenir l'assemblage amovible par emboitement élastique recherché.

La force d'emboitement/déboitement élastique est adaptée pour obtenir un assemblage satisfaisant des deux éléments 25-32 tout en ne nécessitant pas un effort trop important de la part de l'opérateur.

Le câble de haubanage 4 consiste en un câble en titane, acier inoxydable, polyéthylène ou autre. On utilise par exemple un câble d'acier inoxydable de 1 mm de diamètre et dont la longueur est de l'ordre de 50 cm.

La mise en oeuvre de l'équipement 1 selon l'invention sur une fracture de la rotule ou de l'olécrane notamment, commence, après dégagement du champ opératoire, par la réalisation de la réduction et le maintien temporaire des différents fragments osseux à l'aide d'un davier à pointes, puis par la mise en place des broches de maintien 2, 2'.

Pour cette mise en place, après un choix de la longueur idéale, chaque broche 2, 2' est positionnée sur l'outil de manoeuvre 3, lui-même associé à un moteur chirurgical, et elle est insérée au moteur dans le matériau osseux de réception selon un emplacement et une orientation adaptés.

Le vissage est réalisé jusqu'à ce que la butée monobloc 26 des broches de maintien 2, 2' arrive en appui contre le matériau osseux de réception R. Alors, seule la tête 25 des broches de maintien 2, 2' fait saillie hors de ce matériau osseux de réception R.

Le câble de haubanage 4 peut ensuite être posé sur la zone osseuse à traiter, en l'insérant au travers des orifices 252 des différentes broches de maintien 2, 2' positionnées, pour assurer son maintien.
La forme allongée des orifices 252 confère un petit jeu de positionnement, facilitant la pose du câble 4.

Le verrouillage en position du câble de haubanage 4 s'effectue en torsadant ses deux extrémités jusqu'à obtention d'une compression satisfaisante. Les extrémités libres non utilisées du câble 4 sont enfin sectionnées.

Les parties filetées 211, 213 des broches de maintien 2, 2' assurent un ancrage efficace limitant leurs possibilités de recul dans le temps, hors du matériau osseux de réception R.

De plus, les deux parties filetées 213a et 213b des broches de maintien 2', avec des pas de vis différents, et séparées par une âme lisse 21a, permettent au chirurgien de mettre en compression deux parties osseuses séparées par un trait de fracture, pour favoriser la reconstruction osseuse.

La structure de l'outil de manoeuvre 3 permet la mise en place des broches de maintien filetées 2, 2' au sein du matériau osseux de réception, et ses moyens d'assemblage amovible 323 avec la tête 25 des broches de maintien 2, 2' facilitent l'opération correspondante.

Les orifices 252 des têtes de broches de maintien 25 assurent un positionnement précis et stable du câble de haubanage 4.
Le serrage de ce câble de haubanage 4 sur le matériau osseux de réception assure une poussée sur les broches de maintien 2, 2' qui participe à limiter leurs possibilités de recul au sein de ce matériau osseux de réception.
Ce serrage permet aussi de créer de la compression entre les fragments osseux.

La tête 25 des broches de maintien 2, 2' fait saillie du matériau osseux de réception ; et sa forme oblongue avec angles arrondis limite son agressivité vis-à-vis des tissus environnants, une fois le champ opératoire refermé.

On notera que la tête 25 des broches de maintien 2, 2' peut être prévue déformable par écrasement sur le câble de haubanage 4, après la pose de ce dernier, de manière à diminuer encore son caractère saillant et agressif, et aussi pour fixer le positionnement du câble 4 sur le matériau osseux de réception (indépendamment ou en complément de l'opération de torsadage précitée).

Les échancrures 256 peuvent être adaptées pour remplir cette fonction d'écrasement. Si besoin, d'autres zones d'affaiblissement de matière peuvent aussi être prévues ; le choix du matériau peut aussi être adapté pour rendre cet écrasement possible.

L'équipement 1 selon l'invention peut être utilisé pour le traitement de différents types de fracture par haubanage, notamment des fractures de la rotule ou de l'olécrane.

Eventuellement, il peut également être utilisé en tant que broche sans cerclage, car la mise en place est très ergonomique et son filetage assure sa stabilité dans l'os empêchant la principale complication de migration des broches classiques.

Un même équipement 1 peut comporter des broches de maintien 2 ou 2' identiques entre elles.
Il peut aussi comporter des broches de maintien 2 et/ou 2' différentes en dimensions et/ou en structure. Si besoin un même équipement peut comporter à la fois des broches de maintien 2 avec une seule partie filetée 211 et des broches de maintien 2' avec deux parties filetées de pas différent 213a, 213b séparées par une âme lisse 21a.

Dans une variante de réalisation, le corps allongé des broches peut être muni d'un filetage sur toute sa longueur.

Encore selon une autre variante de réalisation, la tête des broches peut comporter une pluralité d'orifices adaptés au passage du câble de haubanage.

## Revendications

1. Equipement pour le traitement d'une fracture par haubanage, par exemple d'une fracture de l'olécrane ou de la rotule, lequel équipement comprend au moins deux broches de maintien (2, 2') associées à un câble de haubanage (4), chaque broche de maintien (2, 2') comportant un corps allongé (21) d'axe longitudinal (L) destiné à être enfoncé dans le matériau osseux de réception (R), lequel corps allongé (21) comporte une extrémité distale (22) qui se termine par une pointe (23), et une extrémité proximale (24) qui se termine par une tête (25),
**caractérisé en ce que** :
- ledit corps allongé (21) desdites broches de maintien (2, 2') comporte au moins une partie (211, 213) munie d'un filetage,
- ladite tête (25) desdites broches de maintien (2, 2') comporte un orifice (252) adapté au passage dudit câble de haubanage (4),
et **en ce que** ledit équipement (1) comprend encore un outil de manoeuvre (3) muni d'une tête de travail (32) agencée pour coopérer avec ladite tête (25) d'une desdites broches de maintien (2, 2'), pour la manoeuvre en rotation de son corps allongé (21) autour dudit axe longitudinal (L), en vue de son ancrage par vissage dans ledit matériau osseux de réception (R) au moyen de ladite partie filetée (211, 213).

2. Equipement selon la revendication 1, **caractérisé en ce que** ladite tête (25) desdites broches de maintien (2, 2') est en forme d'anneau muni d'une embase (251) raccordée de façon monobloc avec l'extrémité proximale (24) dudit corps allongé (21), laquelle tête en forme d'anneau (25) comporte ledit orifice (252) de passage dudit câble de haubanage (4) et est délimitée par deux faces latérales (253), par deux faces d'extrémités (254) et par une face de dessus (255).

3. Equipement selon la revendication 2, **caractérisé en ce que** ladite tête (25) en forme d'anneau est raccordée avec l'extrémité proximale (24) dudit corps allongé (21) par l'intermédiaire d'une structure de butée monobloc (26) pour bloquer son enfoncement dans le matériau osseux de réception (R).

4. Equipement selon la revendication 2, **caractérisé en ce que** ladite tête de travail (32) de l'outil de manoeuvre (3) comporte un logement débouchant (321) destiné à la réception de ladite tête (25) en forme d'anneau de chacune desdites broches de maintien (2, 2'), lequel logement débouchant (321) comprend deux surfaces de côtés (3211 ; 3212), en vis-à-vis, entre lesquelles ladite tête en forme d'anneau (25) est destinée à venir se positionner avec ses faces latérales (253) en regard desdites surfaces de côtés (3211 ; 3212), pour permettre la transmission d'un couple en vue de la manoeuvre en rotation du corps allongé (21) de chacune desdites broches de maintien (2, 2'), pour son ancrage par vissage dans le matériau osseux de réception (R).

5. Equipement selon la revendication 4, **caractérisé en ce que** ladite tête de travail (32) dudit outil de manoeuvre (3) et ladite tête (25) de chacune desdites broches de maintien (2, 2') comportent des moyens complémentaires (323,256) pour leur assemblage amovible.

6. Equipement selon la revendication 5, **caractérisé en ce que** ladite tête de travail (32) dudit outil de manoeuvre (3) et ladite tête (25) de chacune desdites broches de maintien (2, 2') comportent des moyens complémentaires (323,256) pour leur assemblage amovible par emboitement élastique.

7. Equipement selon la revendication 6, **caractérisé en ce que** le logement débouchant (321) de la tête de travail (32) dudit outil de manoeuvre (3) est bordé par une paroi latérale (322) comportant deux fenêtres (325) en vis-à-vis qui s'ouvrent dans ledit logement débouchant (321), pour le positionnement, chacune, d'une partie active (3231) d'un anneau élastique (323) ceinturant ladite paroi latérale (322),
et **en ce que** ladite tête (25) en forme d'anneau de chacune desdites broches de maintien (2, 2') comporte deux échancrures (256) ménagées dans lesdites faces d'extrémités (254), pour la réception desdites parties actives (3231) dudit anneau élastique (323), en fin d'introduction de ladite tête en forme d'anneau (25) dans ledit logement débouchant (321) dudit outil de manoeuvre (3), pour obtenir ledit emboitement élastique amovible.

8. Equipement selon l'une quelconque des revendications 2 à 7, **caractérisé en ce que** l'orifice (252) de passage du câble de haubanage (4) ménagé dans la tête (25) desdites broches de maintien (2, 2') est de forme oblongue pour autoriser un jeu de positionnement pour ledit câble de haubanage (4).

9. Equipement selon l'une quelconque des revendications 1 à 8, **caractérisé en ce que** ladite tête (25) desdites broches de maintien (2, 2') est structurée pour autoriser sa déformation par écrasement sur ledit câble (4).

10. Equipement selon l'une quelconque des revendications 1 à 9, **caractérisé en ce que** ledit corps allongé (21) d'au moins une desdites broches de maintien (2') comporte deux parties filetées (213a, 213b) séparées par une âme lisse (21a), lesquelles deux parties filetées (213a, 213b) comportent un pas de vis différent pour autoriser une mise en compression de deux parties du matériau osseux de réception (R), séparées par une fracture (F).
